Europäisches Patentamt

European Patent Office

Office européen des brevets

(11) Veröffentlichungsnummer : **0 167 643**
**B1**

(12) **EUROPÄISCHE PATENTSCHRIFT**

(45) Veröffentlichungstag der Patentschrift :
15.06.88

(51) Int. Cl.⁴ : **C 12 N    1/04, C 12 N    1/18**

(21) Anmeldenummer : 84107972.6

(22) Anmeldetag : 07.07.84

(54) **Verfahren zur Herstellung von aktiver Trockenhefe mit verbesserter Stabilität.**

(43) Veröffentlichungstag der Anmeldung :
15.01.86 Patentblatt 86/03

(45) Bekanntmachung des Hinweises auf die Patenterteilung : 15.06.88 Patentblatt 88/24

(84) Benannte Vertragsstaaten :
AT BE CH FR GB IT LI LU NL SE

(56) Entgegenhaltungen :
US-A- 3 041 249
US-A- 3 440 058
US-A- 3 440 059

(73) Patentinhaber : Schill & Seilacher GmbH & Co.
Moorfleeterstrasse 28
D-2000 Hamburg 74 (DE)

(72) Erfinder : Tent, Walter
Wiesengrund 10
D-2000 Hamburg 73 (DE)
Erfinder : Feistle, Leo
Mühlenstrasse 49a
D-2056 Glinde (DE)
Erfinder : Boscheinen, Horst
Am alten Sportplatz 14a
D-2105 Seevetal 3 (DE)

(74) Vertreter : Schulmeyer, Karl-Heinz, Dr.
Kieler Strasse 59a
D-2087 Hasloh (DE)

Jouve, 18, rue St-Denis, 75001 Paris, France

**Beschreibung**

Vorliegende Erfindung betrifft ein Verfahren zur Herstellung von aktiver Trockenhefe mit verbesserter Stabilität, bei dem man eine wässrige Suspension von Hefezellen mit einer wässrigen Emulsion eines schützenden Zusatzmittels vermischt.

Aktive Trockenhefen haben gegenüber den herkömmlichen feuchten Preßhefen eine Reihe von Vorteilen, wie erheblich bessere Haltbarkeit und wesentlich geringeres Gewicht und Volumen, so daß sich Trockenhefen leichter transportieren und länger und einfacher lagern lassen. Nachteilig ist hingegen, daß diese Trockenhefen gegenüber Sauerstoff empfindlich sind ; durch Berührung mit dem Sauerstoff der Luft wird die biologische Aktivität der Hefe insbesondere während des Trocknungsvorganges und danach stark beeinträchtigt, so daß trotz zahlreicher Vorkehrungen und Vorsichtsmaßnahmen die Trockenhefe im Vergleich zur Preßhefe stets eine deutlich geringere Triebkraft bzw. Gäraktivität aufweist.

Bei der Verwendung der Hefe in Bäckereien oder im Haushalt ist die Gäraktivität, d. h. die Fähigkeit der Hefe zur Bildung von $CO_2$ aus den im Teig vorhandenen Kohlehydraten, wohl die wichtigste Eigenschaft der Hefe.

Der Hefetrocknungsprozeß wird üblicherweise bei einer Temperatur von ca. 30-35 °C durchgeführt und kann in Verbindung mit der intensiven Belüftung eine gesteigerte Atmung der Hefezellen verursachen. Dabei werden die in den Zellen enthaltenen Reservesubstanzen veratmet, wodurch die biologische Aktivität der Trockenhefe stark reduziert wird. Ein weiterer Verlust an Gäraktivität bzw. an Triebkraft wird offenbar durch die morphologischen Veränderungen der Hefezellen verursacht, die während des Trocknungsprozesses durch die Entfernung von extrazellulärem und intrazellulärem Wasser hervorgerufen werden. Dadurch erfolgt eine Schrumpfung der Zellwand, die dadurch spröde wird und einreißt. Elektronenmikroskopische Aufnahmen zeigen deutlich, daß die bei der Trocknung beschädigten Zellwände zum größten Teil bei der Rehydratisierung die ursprüngliche Form nicht wieder annehmen, also abgestorben sind.

Man hat deshalb versucht, die Einbuße an Gäraktivität durch besondere Vorsichtsmaßnahmen bei der Trocknung möglichst gering zu halten, beispielsweise dadurch, daß man die Preßhefe auf dem schnellsten Wege bis zur Trockenhefe aufgearbeitet und dabei die Temperatur der Hefesuspension möglichst niedrig gehalten hat, um Substanzverluste durch die Atmung der lebenden Hefe in Grenzen zu halten.

Da trotz solcher Vorsichtsmaßnahmen die Aktivitätsverluste der erhaltenen Trockenhefen im Vergleich zur Aktivität der feuchten Preßhefe noch beträchtlich sind, hat man ferner bereits versucht, durch Zusatz geeigneter Schutzstoffe zu den zu trocknenden Hefesuspensionen eine Trockenhefe mit besserer Stabilität und insbesondere besserer Gäraktivität zu erhalten.

So wird in US-PS 2 894 842 vorgeschlagen, der Hefesuspension vor der Trocknung einen Sorbitanfettsäureester zuzusetzen. Die Hefe wird bis auf einen Feuchtigkeitsgehalt von weniger als 6 % getrocknet. Die resultierende Trockenhefe soll direkt in warmen Wasser rehydratisierbar sein. Trotz des geringen Feuchtigkeitsgehaltes ist die Stabilität der Trockenhefe jedoch gegenüber Luftsauerstoff nicht ausreichend, so daß auch in diesem Falle wie üblich die Trockenhefe unter Stickstoffatmosphäre oder Vakuum verpackt werden muß. Außerdem sind für das in der zitierten US-Patentschrift beschriebene sogenannte « Spaghetti »-Verfahren nur bestimmte Preßhefen mit einem niedrigen Protein- und Phosphorgehalt geeignet, da andere Hefearten unter den Trocknungsbedingungen des bekannten Verfahrens Trockenhefen ergeben, deren Triebkraft häufig nicht mehr für eine Backhefe ausreicht.

Aus GB-PS 1 132 793 ist ein ähnliches Verfahren zur Herstellung von Trockenbackhefe mit einem Feuchtigkeitsgehalt von 8 % beschrieben, bei dem der Preßhefe vor der Trocknung 0,1 bis 5 % eines Zusatzmittels, nämlich eines Monoglycerids, Sorbitanfettsäureesters oder Maisöl, zugesetzt werden. Anschließend wird das Gemisch granuliert und mit 40 °C warmer Luft im Fließbettverfahren innerhalb von 1 bis 2 Stunden getrocknet. Auch die nach diesem Verfahren erhaltene Trockenhefe ist deutlich weniger aktiv als Preßhefe.

In DE-AS 1 261 101 wird ein Verfahren zur Herstellung von aktiver Trockenhefe beschrieben, bei dem die wäßrige Hefezellensuspension mit einer wäßrigen Emulsion vermischt wird, die butyliertes Hydroxyanisol, butyliertes Hydroxytoluol oder Propylgallat, eßbares Fett, eßbares Öl oder Propylenglykol und ein nichtgiftiges, nichtionisches oberflächenaktives Mittel z. B. Sorbitanmonostearat enthält.

Durch diese bekannte Kombination eines Antioxydationsmittels mit einem oberflächenaktiven Mittel oder Emulgiermittel kann zwar eine gewisse Stabilität des Trockenhefeproduktes erzielt werden, die Triebkraft liegt jedoch auch weiterhin erheblich unter derjenigen einer vergleichbaren Menge an Preßhefe, so daß eine weitere Verbesserung der Eigenschaften der aktiven Trockenhefe wünschenswert erscheint. Im übrigen haben Vergleichsversuche ergeben, daß sich die in der DE-AS 1 261 101 vorgeschlagenen Emulsionskomponenten, wenn überhaupt, nur sehr unvollständig zu einer Emulsion verarbeiten lassen, so daß dieses Verfahren offenbar schwierig durchzuführen ist.

Die US-A-3 440 058 beschreibt ein ähnliches Verfahren, worin die Emulsion Zitronensäureester von Stearinmonoglyceriden oder Diacetylweinsäureester von eßbaren Fettsäuremonoglyceriden enthält.

Ferner ist ein Verfahren zur Herstellung von aktiver Bäckerhefe aus DE-OS 2 117 901 bekannt, bei

2

dem man von ausgesuchten Mutanten von Bäckerhefe ausgeht, die sich zu Trockenhefen hoher Aktivität verarbeiten lassen sollen, wobei der Hefe vor dem Trocknungsprozeß vorzugsweise Quellmittel und/oder Benetzungsmittel zugemischt werden, u. a. Mono- und Diglyceride eßbarer Fettsäuren, die mit organischen Säuren, wie Zitronensäure oder Weinsäure, modifiziert sind. Abgesehen davon, daß dieses Verfahren offenbar nur für bestimmte Hefemutanten geeignet ist, haben Vergleichsversuche ergeben, daß der Zusatz der genannten Quell- und Benetzungsmittel nicht verhindern kann, daß die Hefe während des Trocknungsprozesses einen erheblichen Teil ihrer Gäraktivität im Vergleich zur frischen Preßhefe einbüßt.

Der vorliegenden Erfindung liegt daher die Aufgabe zugrunde, ein Verfahren zur Herstellung von aktiver Trockenhefe zu schaffen, bei dem durch Verwendung geeigneter Zusatzmittel, die die Hefezellen insbesondere während des Trocknungsprozesses wirksam vor Schädigungen schützen, aktive Trockenhefen erhalten werden, die eine verbesserte Stabilität und insbesondere eine stabilisierte Aktivität aufweisen, so daß die Triebkraft der erhaltenen Trockenhefen über einen längeren Zeitraum praktisch in vollem Umfang erhalten bleibt und bei etwa 90 % der Triebkraft einer entsprechenden Menge Preßhefe liegt.

Diese Aufgabe wird erfindungsgemäß gelöst durch ein Verfahren zur Herstellung von aktiver Trockenhefe mit verbesserter Stabilität, bei dem man eine wäßrige Suspension von Hefezellen mit einer wäßrigen Emulsion eines schützenden Zusatzmittels vermischt, anschließend das überschüssige Wasser entfernt, die erhaltene feuchte Hefemasse mit einem Feuchtigkeitsgehalt von etwa 65 bis 70 Gew.% in kleine Stücke zerteilt und diese in herkömmlicher Weise bis auf einen Feuchtigkeitsgehalt von 8 % oder weniger trocknet. Das erfindungsgemäße Verfahren ist dadurch gekennzeichnet, daß man als schützendes Zusatzmittel in einer Menge von 0,5 bis 2 Gew.-%, bezogen auf die Hefetrockensubstanz, eine wäßrige Emulsion einer Mischung aus

20-80 Gewichtsteilen Speisefett mit einer Jodzahl von höchstens 10 und
8-55 Gewichtsteilen Zitronensäureester von eßbaren Fettsäuremono- und/oder -diglyceriden und
2-50 Gewichtsteilen Diacetylweinsäureester eßbarer Fettsäuremonoglyceride und
12-72 Gewichtsteilen Sorbitanmonostearat und/oder
1-15 Gewichtsteilen Sulfobernsteinsäurediester und/oder
1-15 Gewichtsteilen butyliertem Hydroxytoluol

einsetzt mit der Maßgabe, daß das Zusatzmittel aus mindestens vier Komponenten besteht.

Überraschenderweise wurde gefunden, daß die erfindungsgemäßen Kombinationen von Schutzstoffen, angewandt in Form einer wäßrigen Emulsion, eine erheblich bessere stabilisierende Wirkung auf die Gäraktivität der resultierenden aktiven Trockenhefe zeigen als bisher bekannte Zusatzmittel.

Die einzelnen Komponenten des erfindungsgemäß eingesetzten Zusatzmittels sind sämtlich bekannte, als Zusatz für Lebensmittel zugelassene Produkte. Die Basis des erfindungsgemäßen Zusatzmittels besteht aus einer Mischung aus einem Speisefett pflanzlichen oder tierischen Ursprungs mit einer Jodzahl von höchstens 10, vorzugsweise mit einer Jodzahl von weniger als 5, besonders bevorzugt aus einem entsprechenden hydrierten Speisefett und ganz besonders bevorzugt aus einem Speisefett mit einer Jodzahl von nicht mehr als 2, einem Zitronensäureester von eßbaren Fettsäuremono- und/oder -diglyceriden, vorzugsweise von Stearin- und/oder Palmitinsäuremono- und/oder -diglyceriden, sowie einem Diacetylweinsäureester von verschiedenen eßbaren Fettsäuremonoglyceriden, zum Beispiel von Stearinsäuremonoglyceriden. Zu dieser Basismischung wird mindestens noch eine weitere Komponente aus der Gruppe Sulfobernsteinsäurediester, bevorzugt Sulfobernsteinsäuredioctylester, butyliertes Hydroxytoluol und/oder Sorbitanmonostearat zugefügt, so daß sich das erfindungsgemäße Zusatzmittel aus mindestens vier Bestandteilen zusammensetzt.

Besonders bewährt hat sich der Einsatz eines Zusatzmittels aus wenigstens 25 Gewichtsteilen Speisefett mit einer Jodzahl von höchstens 10, 30 bis 52 Gewichtsteilen Zitronensäureester von eßbaren Fettsäuremono- und/oder -diglyceriden, 5 bis 20 Gewichtsteilen Diacetylweinsäureester eßbarer Fettsäuremonoglyceride, 5 bis 10 Gewichtsteilen Sulfobernsteinsäurediester und 2 bis 8 Gewichtsteilen butyliertem Hydroxytoluol, weshalb diese Zusammensetzung des Zusatzmittels in dem erfindungsgemäßen Verfahren besonders bevorzugt wird.

In einer weiteren bevorzugten Ausführungsform des erfindungsgemäßen Verfahrens besteht das eingesetzte Zusatzmittel aus 20 bis 40 Gewichtsteilen Speisefett der vorstehend angegebenen Art, 25 bis 45 Gewichtsteilen Zitronensäureester von eßbaren Fettsäuremono- und/oder -diglyceriden, 5 bis 30 Gewichtsteilen Diacetylweinsäureester eßbarer Fettsäuremonoglyceride und 25 bis 45 Gewichtsteilen Sorbitanmonostearat.

Beim erfindungsgemäßen Verfahren geht man von einem Ausgangsmaterial aus, das aus einer wäßrigen Suspension von Hefezellen, zum Beispiel von Saccharomyces cerevisiae, hergestellt und als « Heferahm » mit beispielsweise einer Dichte entsprechend 17° Balling eingesetzt wird. Gemäß der Erfindung wird zu dieser Suspension eine wäßrige Emulsion des schützenden Zusatzmittels zugefügt, und zwar zweckmäßig in einer Menge von 0,5 bis 2 Gew.%, vorzugsweise in einer Menge von 0,8 bis 1,5 Gew.%, bezogen auf die Hefetrockensubstanz. Die Herstellung der wäßrigen Emulsion erfolgt in einer dem Fachmann bekannten Weise, wobei gegebenenfalls die Mischung der Komponenten auf eine

3

erhöhte Temperatur, zum Beispiel auf 50 bis 60 °C, erwärmt und vor der Zugabe zu der Hefesuspension wieder auf Raumtemperatur abgekühlt wird. Dabei liegt die Mischung aus Antioxydans und Speisefett als disperse Phase und das Wasser als Kontinuierliche Phase vor.

Unter mechanischem Rühren wird die Suspension in Bewegung gehalten und das zugemischte Zusatzmittel homogen verteilt. Dabei kann die Temperatur in dem Rühr- oder Mischwerk im Bereich von ca. 4 °C bis Raumtemperatur liegen.

Anschließend wird diese Hefesuspension mit Hilfe einer Filtervorrichtung abfiltriert und der erhaltene Filterkuchen, zum Beispiel durch eine Presse, bis auf einen Feuchtigkeitsgehalt von etwa 65 bis 70 Gew.% entwässert. Die erhaltene, so entwässerte Naßhefe wird dann in an sich bekannter Weise in kleine Stücke zerteilt, beispielsweise mit einem Granulator granuliert oder mit Hilfe einer Strangpresse in Form von Bändern oder « Nudeln » ausgepreßt oder mit einer anderen geeigneten Vorrichtung in Teilchen der gewünschten Form und Größe gebracht. Bevorzugt werden wegen ihrer guten Handhabbarkeit Teilchen in Form von Granulaten, beispielsweise mit einem Durchmesser von 0,4 bis 0,8 mm und einer Länge von 3 bis 6 mm.

Die erhaltenen Hefeteilchen werden anschließend unter Anwendung herkömmlicher Techniken schonend getrocknet, beispielsweise in einem Wirbelschichttrockner, wobei die Zulufttemperatur bei Beginn des Trocknens zwischen etwa 50 und 80 °C und am Ende des Trocknens zwischen etwa 30 und 40 °C liegt und die Restfeuchtigkeit von ca. 65 auf ca. 8 % oder weniger erniedrigt wird. Die Trocknungszeit schwankt hierbei je nach Menge der zu trocknenden Hefesubstanz und den weiteren angewandten Bedingungen zwischen einer halben Stunde bis etwa zwei Stunden.

Der Feuchtigkeitsgehalt der nach dem erfindungsgemäßen Verfahren hergestellten fertigen aktiven Trockenhefe liegt in der Regel zwischen 6 und 8 %, gewünschtenfalls aber auch niedriger. Die fertige granulierte Trockenhefe ist hell gefärbt, gießbar, nicht klebrig und nicht staubig und läßt sich gut dosieren. Durch elektronenmikroskopische Aufnahmen der erfindungsgemäß hergestellten aktiven Trockenhefe konnte festgestellt werden, daß die Hefezellen nach dem Trocknungsprozeß im wesentlichen gut erhalten waren, was offensichtlich auf den günstigen Einfluß der erfindungsgemäß eingesetzten schützenden Zusatzmittel zurückzuführen ist.

Dementsprechend zeigen die nach dem erfindungsgemäßen Verfahren hergestellten aktiven Trockenhefen eine ausgezeichnete Gäraktivität, die in der Regel bei 90° der Gäraktivität der entsprechenden Preßhefe und in besonders günstigen Fällen sogar noch höher liegt. Diese gute Gäraktivität bleibt auch bei längerer Lagerung unter Normalbedingungen im wesentlichen erhalten. In Versuchen wurde festgestellt, daß nach einjähriger Lagerung unter Stickstoffatmosphäre bei Raumtemperatur die Gäraktivität der erfindungsgemäß hergestellten aktiven Trockenhefe um etwa 15 bis 20 % niedriger lag als die Anfangsaktivität.

Die Erfindung wird anhand der folgenden Beispiele weiter erläutert.

Beispiel 1

Zu 700 g einer direkt aus einem Backhefeproduktionsbetrieb stammenden Preßhefe der Gattung Saccharomyces cerevisiae mit einem Feuchtigkeitsgehalt von etwa 72 %, die mit 4 850 ccm Wasser zu einem « Heferahm » mit ca. 17° Balling suspendiert worden war, wurden 3,5 g eines erfindungsgemäßen Zusatzmittels, das in 40 ccm Wasser mit Hilfe eines handelsüblichen Homogenisators bei einer Temperatur von ca. 50 bis 55 °C emulgiert worden war, nach dem Abkühlen auf Raumtemperatur zugefügt. Das Zusatzmittel besaß folgende Zusammensetzung :

25 Gewichtsteile hydrierter Talg, Jodzahl 1,5 ;

28 Gewichtsteile Zitronensäureester von Stearin- und Palmitinsäuremono- und -diglyderiden, Säurezahl ca. 35, Verseifungszahl 220 bis 250, Hydroxylzahl 215 bis 240, Jodzahl kleiner als 2 ;

28 Gewichtsteile Sorbitanmonostearat, Steigschmelzbereich 50 bis 53 °C ;

15 Gewichtsteile Diacetylweinsäureester eßbarer Fettsäure monoglyceride, Schmelzbereich 42 bis 49 °C, Säurezahl 85 bis 105, Verseifungszahl 460 bis 520, Jodzahl kleiner als 2 ;

2 Gewichtsteile Sulfobernsteinsäuredioctylester, Säurezahl max. 3 ;

2 Gewichtsteile butyliertes Hydroxytoluol, Schmpkt. 67 °C.

Die Mischung aus Zusatzmittelemulsion und Hefesuspension wurde unter mechanischem Rühren 30 Minuten lang bei 300 U/Min. gründlich durchmischt und in ständiger Bewegung gehalten. Danach wurde die Suspension über ein Büchner-Filter abfiltriert. Der Filterkuchen wurde mit Hilfe einer Presse bis auf einen Feuchtigkeitsgehalt von ca. 65 % abgepreßt.

Anschließend wurde die Hefe mit einer handelsüblichen Strangpressvorrichtung (sog. Granulator) in der Weise granuliert, daß die Granulate einen Durchmesser von etwa 0,6 mm und eine Länge von ca. 5 mm aufwiesen. Diese Granulate wurden in einem Wirbelschichttrockner (Labortrockner, Firma Aeromatik AG, Schweiz) etwa 30 Minuten lang getrocknet, wobei die Temperatur der Zuluft zu Beginn des Trocknens bei etwa 80 °C lag und während des Trocknungsvorganges allmählich bis auf ca. 32 °C abgesenkt wurde. Das getrocknete Hefeprodukt besaß noch eine Restfeuchtigkeit von ca. 4,5 %.

In einem Vergleichsversuch wurde eine entsprechende Hefeprobe ohne Zusatzmittel in der gleichen Weise, wie oben beschrieben, behandelt und getrocknet.

Das nach dem erfindungsgemäßen Verfahren erhaltene Trockenhefeprodukt bestand aus leicht fließfähigen, nicht klebrigen Körnchen von hellgelber Farbe, während das im Vergleichsversuch ohne schützendes Zusatzmittel hergestellte Trockenhefeprodukt durch eine dunkle gelbbraune Färbung bereits optisch leicht von der erfindungsgemäß hergestellten Trockenhefe unterschieden werden konnte.

Die Gäraktivität (Triebkraft) der erhaltenen aktiven Trockenhefe wurde nach einem standardisierten Prüfverfahren wie folgt ermittelt :

2,25 g der aktiven Trockenhefe wurden zusammen mit 5 g Zucker in 55 ccm Wasser von 30 °C 5 Minuten lang rehydratisiert und dann zu einem Standardteig zugefügt, der aus 300 g Mehl, 120 ccm Wasser und 7,5 g Salz hergestellt worden war. Das Ganze wurde innig miteinander vermischt. Anschließend wurde die Triebkraft dieses Teiges in einem standardisierten S.J.A.-Fermentografen folgendermaßen gemessen :

Man ließ den Teig 60 Minuten lang bei 30 °C treiben, knetete ihn danach durch und ließ ihn erneut 60 Minuten lang bei 30 °C treiben. Die entwickelte Menge an $CO_2$ (in ccm) wurde während des gesamten Treibvorganges automatisch gemessen und die gemessenen $CO_2$-Werte kontinuierlich von einem Schreiber aufgezeichnet.

Die Treibversuche ergaben, daß die nach dem erfindungsgemäßen Verfahren hergestellte aktive Trockenhefe eine Triebkraft besaß, die etwa 90 % der Triebkraft der entsprechenden Ausgangspreßhefe entspricht. Die Vergleichsprobe ohne schützenden Zusatz zeigte hingegen nur eine Triebkraft, die etwa 40 % derjenigen der Ausgangspreßhefe entspricht.

## Beispiel 2

Beispiel 1 wurde wiederholt mit dem Unterschied, daß zum Heferahm 3,9 g eines in 40 ccm Wasser emulgierten Zusatzmittels der folgenden Zusammensetzung zugefügt wurden :

43 Gewichtsteile Kokosfett, Jodzahl 8 ;
38 Gewichtsteile Zitronensäureester (wie in Beispiel 1) ;
12 Gewichtsteile Diacetylweinsäureester (wie in Beispiel 1) ;
5 Gewichtsteile Sulfobernsteinsäuredioctylester, Säurezahl max. 3 ;
2 Gewichtsteile butyliertes Hydroxytoluol, Schmelzpunkt 67 °C.

Das getrocknete Hefeprodukt besaß noch eine Restfeuchtigkeit von ca. 4 % und bestand aus leicht fließfähigen, nicht klebrigen Körnchen von hellgelber Farbe. Die Triebkraft wurde, wie in Beispiel 1 beschrieben, gemessen und ergab etwa 90 % der Triebkraft der entsprechenden Ausgangspreßhefe.

## Beispiel 3

Beispiel 1 wurde wiederholt mit dem Unterschied, daß zum Heferahm 3,5 g eines in 40 ccm Wasser emulgierten Zusatzmittels der folgenden Zusammensetzung zugefügt wurden :

25 Gewichtsteile hydrierter Talg, Jodzahl 1,5 ;
30 Gewichtsteile Zitronensäureester (wie in Beispiel 1) ;
15 Gewichtsteile Diacetylweinsäureester (wie in Beispiel 1) ;
30 Gewichtsteile Sorbitanmonostearat (wie in Beispiel 1).

Das getrocknete Hefeprodukt besaß noch eine Restfeuchtigkeit von ca. 4,5 %. Das Produkt zeichnete sich durch zylindrisch geformte Teilchen mit einer mittleren Teilchengröße von 0,5 × 0,7 mm mit glatter, schwach glänzender Oberfläche aus. Diese erfindungsgemäß hergestellte aktive Trockenhefe bestand aus leicht fließfähigen, nicht klebrigen Körnchen von hellgelber Farbe und neutralem Geruch.

Zu Vergleichszwecken wurde außerdem in gleicher Weise, wie in Beispiel 1 beschrieben, eine Trockenhefe mit einem herkömmlichen Zusatzmittel, nämlich einem Zitronensäureester von Stearin- und Palmitinsäuremono- und -diglyceriden, hergestellt. Das getrocknete Produkt besaß noch einen Feuchtigkeitsgehalt von ca. 6 % und bestand aus hellgelb gefärbten, frei fließenden Körnchen.

Die Prüfung der Gäraktivität sowohl der nach dem erfindungsgemäßen Verfahren hergestellten aktiven Trockenhefe als auch des mit dem herkömmlichen Zusatzmittel versehenen Trockenhefeproduktes erfolgte in der in Beispiel 1 beschriebenen Weise.

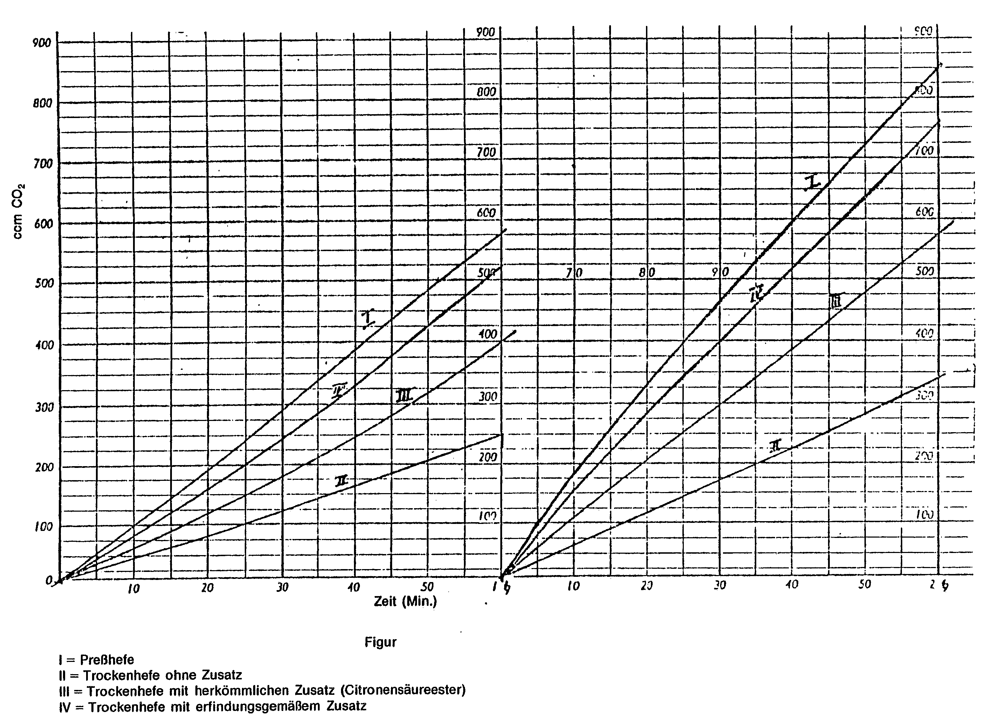

Die Ergebnisse sind in der beigefügten Figur grafisch dargestellt. Es zeigen :

Kurve I : feuchte Preßhefe
Kurve II : aktive Trockenhefe ohne Zusatz
Kurve III : aktive Trockenhefe mit einem üblichen Zusatz (Zitronensäureester von eßbaren Fettsäuremono- und -diglyceriden)
Kurve IV : aktive Trockenhefe mit erfindungsgemäßem Zusatz gemäß Beispiel 3.

Während die Triebkraft der erfindungsgemäß hergestellten Trockenhefe bei etwa 90 % der Triebkraft der Ausgangspreßhefe liegt (Kurve IV im Vergleich zu Kurve I), beträgt die Triebkraft der Vergleichsprobe (Kurve III) nur etwa 69 % und der Trockenhefe ohne Zusatzmittel (Kurve II) sogar nur etwa 40 % der Triebkraft der Ausgangspreßhefe.

Die nach dem erfindungsgemäßen Verfahren hergestellte aktive Trockenhefe zeigt demnach eine erheblich bessere Triebkraft als eine Trockenhefe, die mit einem herkömmlichen Zusatzmittel ausgerüstet worden ist, und kommt in ihrer Gäraktivität ziemlich nahe an die Ausgangspreßhefe heran. In weiteren gezielten Versuchen konnte die Triebkraft sogar noch deutlich über 90 % angehoben werden.

Ein weiterer Vorteil der erfindungsgemäß hergestellten aktiven Trockenhefe besteht darin, daß sie sowohl in rehydratisierter Form als auch unmittelbar dem Teig zugemischt werden kann, wobei die Triebkraft in beiden Anwendungsformen praktisch gleich gut ist, wie aus den in der nachfolgenden Tabelle zusammengestellten Ergebnissen entsprechender Treibversuche hervorgeht, bei denen die Triebkraft, wie in Beispiel 1 beschrieben, gemessen wurde.

Tabelle : Triebkraft von erfindungsgemäß hergestellter aktiver Trockenhefe gemäß Beispiel 3

| Zeit (Min.) | Triebkraft von Trockenhefe, | |
|---|---|---|
| | rehydratisiert vor Zusatz zum Standard-teig (in ccm $CO_2$) | unmittelbar dem Standardteig zuge-mischt (in ccm $CO_2$) |
| 30 | 250 | 240 |
| 60 | 525 | 475 |
| 90 | 925 | 975 |
| 120 | 1275 | 1325 |

**Patentansprüche**

1. Verfahren zur Herstellung von aktiver Trockenhefe mit verbesserter Stabilität, bei dem man eine wäßrige Suspension von Hefezellen mit einer wäßrigen Emulsion eines schützenden Zusatzmittels vermischt, anschließend das überschüssige Wasser entfernt, die erhaltene feuchte Hefemasse mit einem Feuchtigkeitsgehalt von etwa 65 bis 70 Gew.% in kleine Stücke zerteilt und diese in herkömmlicher Weise bis auf einen Feuchtigkeitsgehalt von 8 % oder weniger trocknet, dadurch gekennzeichnet, daß man als schützendes Zusatzmittel in einer Menge von 0,5 bis 2 Gew.%, bezogen auf die Hefetrockensubstanz, eine wäßrige Emulsion einer Mischung aus

20 bis 80 Gewichtsteilen Speisefett mit einer Jodzahl von höchstens 10 und
8 bis 55 Gewichtsteilen Zitronensäureester von eßbaren Fettsäuremono- und/oder -diglyceriden und
2 bis 50 Gewichtsteilen Diacetylweinsäureester eßbarer Fettsäuremonoglyceride und
12 bis 72 Gewichtsteilen Sorbitanmonostearat und/oder
1 bis 15 Gewichtsteilen Sulfobernsteinsäurediester und/oder
1 bis 15 Gewichtsteilen butyliertem Hydroxytoluol

einsetzt mit der Maßgabe, daß das Zusatzmittel aus mindestens vier Komponenten besteht.

2. Verfahren nach Anspruch 1, dadurch gekennzeichnet, daß als Speisefett ein hydriertes Fett mit einer Jodzahl von nicht mehr als 2 eingesetzt wird.

3. Verfahren nach Anspruch 1 oder 2, dadurch gekennzeichnet, daß ein Zitronensäureester von Stearin- und/oder Palmitinsäuremono- und/oder -diglyceriden eingesetzt wird.

4. Verfahren nach einem der Ansprüche 1 bis 3, dadurch gekennzeichnet, daß als Sulfobernsteinsäurediester Sulfobernsteinsäuredioctylester eingesetzt wird.

5. Verfahren nach den Ansprüchen 1 bis 4, dadurch gekennzeichnet, daß ein Zusatzmittel aus wenigstens 25 Gewichtsteilen Speisefett,

30 bis 52 Gewichtsteilen Zitronensäureester von eßbaren Fettsäuremono- und/oder -diglyceriden,
5 bis 10 Gewichtsteilen Sulfobernsteinsäurediester,
5 bis 20 Gewichtsteilen Diacetylweinsäureester eßbarer Fettsäuremonoglyceride und
2 bis 8 Gewichtsteilen butyliertem Hydroxytoluol

eingesetzt wird.

6

6. Verfahren nach den Ansprüchen 1 bis 4, dadurch gekennzeichnet, daß ein Zusatzmittel aus

20 bis 40 Gewichtsteilen Speisefett,
25 bis 45 Gewichtsteilen Zitronensäureester von eßbaren Fettsäuremono- und/oder -diglyceriden,
25 bis 45 Gewichtsteilen Sorbitanmonostearat und
5 bis 30 Gewichtsteilen Diacetylweinsäureester eßbarer Fettsäuremonoglyceride

eingesetzt wird.

## Claims

1. Process for the production of active dry yeast with improved stability in which an aqueous suspension of yeast cells is mixed with an aqueous emulsion of a protective additive, after which the excess water is eliminated, the moist yeast mass with a moisture content of some 65 % to 70 % by weight is divided into small pieces and these are dried to a moisture content of 8 % or less, characterized in that as protective additive, in a quantity of 0.5 to 2 % by weight, referred to the dry yeast substance, an aqueous emulsion of a mixture of

20-80 parts by weight of nutrient fat with an iodine number of 10 maximum,
8-55 parts by weight of citric acid esters of edible fatty-acid mono- and/or diglycerides and
2-50 parts by weight of diacetyltartaric acid esters of edible fatty-acid monoglycerides and
12-72 parts by weight of sorbitanmonostearate and/or
1-15 parts by weight of sulphosuccinic acid diester and/or
1-15 parts by weight of butylated hydroxytoluol

is used, with the condition that the additive is made up from at least four components.

2. Process according to Claim 1, characterized in that as edible fat, a hydrated fat with an iodine number of not more than 2 is used.

3. Process according to Claim 1 or 2, characterized in that a citric acid ester of stearic- und/or palmitic acid mono- and/or diglycerides is used.

4. Process according to one of the Claims 1 to 3, characterized in that as sulphosuccinic acid diester, sulphosuccinic acid dioctyl ester is used.

5. Process according to the Claims 1 to 4, characterized in that an additive is used out of at least 25 parts by weight of nutrient fat,

30-52 parts by weight of citric acid esters of edible fatty-acid mono- and/or diglycerides,
5-10 parts by weight of sulphosuccinic acid diester,
5-20 parts by weight of diacetyltartaric acid esters of edible fatty-acid monoglycerides and
2- 8 parts by weight of butylated hydroxytoluol.

6. Process according to the Claims 1 to 4, characterized in that an additive is used out of

20-40 parts by weight of nutrient fat,
25-45 parts by weight of citric acid esters of edible fatty-acid mono- and/or diglycerides,
25-45 parts by weight of sorbitanmonostearate and
5-30 parts by weight of diacetyltartaric acid esters of edible fatty-acid monoglycerides.

## Revendications

1. Procédé de préparation d'une levure séchée active d'une stabilité améliorée, dans lequel on mélange une suspension aqueuse de cellules de levure avec une émulsion aqueuse d'un additif protecteur, on élimine l'eau en excès, on subdivise la masse de levure humide, d'une teneur en humidité d'environ 65 à 70 % en poids, en petits morceaux, qui sont ensuite séchés de façon usuelle jusqu'à une teneur en humidité de 8 % ou moins, caractérisé en ce que l'on utilise comme additif protecteur une émulsion aqueuse d'un mélange comprenant au moins quatre des composants ci-après :

20 à 80 parties en poids d'huile comestible avec un indice d'iode ne dépassant pas 10,
8 à 55 parties en poids de citrates de mono- et(ou) de di-glycérides comestibles d'acides gras,
2 à 50 parties en poids de diacétyl-tartrates de monoglycérides comestibles d'acides gras,
12 à 72 parties en poids de mono-stéarate de sorbitan et(ou)
1 à 15 parties en poids de di-ester sulfo-succinique et(ou)
1 à 15 parties en poids d'hydroxy-toluène butylé,

incorporé à raison de 0,5 à 2 % du poids de la matière sèche de la levure.

2. Procédé suivant la revendication 1, caractérisé en ce que l'huile comestible est une huile hydrogénée avec un indice d'iode ne dépassant pas 2.

3. Procédé suivant la revendication 1 ou 2, caractérisé en ce que l'on utilise un citrate de mono- et(ou) de di-glycérides de l'acide stéarique et(ou) de l'acide palmitique.

4. Procédé suivant l'une des revendications 1 à 3, caractérisé en ce que le diester sulfo-succinique est le sulfo-succinate de dioctyle.

5. Procédé suivant les revendications 1 à 4, caractérisé en ce que l'additif incorporé est composé de au moins 25 parties en poids d'huile comestible,

30 à 52 parties en poids de citrates de mono- et(ou) de di-glycérides comestibles d'acides gras,
5 à 10 parties en poids de di-ester sulfo-succinique,
5 à 20 parties en poids de diacétyl-tartrate de monoglycérides comestibles d'acides gras et
2 à 8 parties en poids d'hydroxy-toluène butylé.

6. Procédé suivant les revendications 1 à 4, caractérisé en ce que l'additif incorporé est composé de

20 à 40 parties en poids d'huile comestible,
25 à 45 parties en poids de citrates de mono- et(ou) de di-glycérides comestibles d'acides gras,
25 à 45 parties en poids de mono-stéarate de sorbitan et
5 à 30 parties en poids de diacétyl-tartrate de monoglycérides comestibles d'acides gras.

ccm CO₂

Zeit (Min.)

Figur

I = Preßhefe

II = Trockenhefe ohne Zusatz

III = Trockenhefe mit herkömmlichen Zusatz (Citronensäureester)

IV = Trockenhefe mit erfindungsgemäßem Zusatz

1